# EUROPEAN PATENT SPECIFICATION

(11) **EP 4 611 698 B1**
(45) Date of publication and mention of the grant of the patent: **13.05.2026**
(21) Application number: 23805125.4
(22) Date of filing: 01.11.2023
(51) Int. Cl.: A61F 5/44, A61F 5/448

(54) **A BASEPLATE FOR AN OSTOMY APPLIANCE**
GRUNDPLATTE FÜR EINE STOMAVORRICHTUNG
PLAQUE DE BASE POUR UN APPAREIL DE STOMIE

(30) Priority: 03.11.2022 US 202263422080 P; 29.11.2022 GB 202217932
(43) Date of publication of application: 10.09.2025
(73) Proprietor: ConvaTec Limited, Deeside, Flintshire CH5 2NU (GB)
(72) Inventor: SAVITSKI, Alexander, Palm Beach Gardens, Florida 33410 (US); EVANS, Jennifer, Deeside Flintshire CH5 2NU (GB); ELLIS, Matt, Deeside Flintshire CH5 2NU (GB)
(74) Representative: Wilson Gunn
(86) International application number: PCT/GB2023/052851
(87) International publication number: WO 2024/094998

(56) References cited:
- US-A1- 2004 089 640
- US-A1- 2009 216 208

## Description

### Technical Field of the Invention

The present invention relates to ostomy appliances and methods of manufacturing an ostomy appliance. In particular, the present invention relates to a baseplate configured to secure an ostomy appliance to the body of a user and most particularly to baseplates adapted for use with a recessed stoma, overweight user and/or stomas with complications such as scar tissue, as well as methods of manufacturing said baseplates.

### Background to the Invention

When using an ostomy appliance, it is paramount that the ostomy appliance is securely attached to the user's body around the stoma in a manner that it can efficiently receive and collect stomal output. It is particularly important that leaks of liquid and/or gas are prevented/minimised as any leaking can cause discomfort or injury, such as skin damage around the stoma, and presents a hygienic risk to the user as well as potential embarrassment and inconvenience.

Typically, an ostomy appliance comprises a pouch in fluid communication with the stoma via an adhesive collar that attaches the ostomy appliance to the skin of the user around the stoma. Conventionally, the collar is attached to the pouch directly via an adhesive or weld, such as a heat weld or ultra-sonic weld. However, in certain circumstances, it is beneficial to control the shape of the collar to enhance transport of stomal output into the pouch and provide a better seal between the user and the ostomy appliance. To achieve this, a shaped-disc that is more rigid than the collar or pouch is attached to the collar to form a baseplate which can then be attached to the pouch - for example a convex disc may be provided for users with a recessed stoma.

An exemplary baseplate/wafer incorporating a convex layer is disclosed in WO2020/220026. In that disclosure, the convex layer is an internal layer i.e. arranged such that one side abuts the skin of the patient in use, and is attached on its opposite side to an external layer, which in turn is coupled to the ostomy pouch. The convex layer of WO2020/220026 is fairly complex, with perforations though which liquid adhesive seeps to attach the wafer to the user.

It would be more straightforward for the internal layer (i.e. that which abuts the patient in use) to be a conventional adhesive collar, with the convex disc provided externally of the adhesive collar and between the adhesive collar and the pouch. The convex disc could then provide a shape that the adhesive collar conforms to.

Unfortunately, use of a shaped-disc (such as the convex layer in WO2020/220026) between the adhesive collar and the pouch could lead to poor performance of the ostomy appliance due the shape of the disc increasing the likelihood of leaks between the different components of the ostomy appliance. For example, if ultrasonic or heat welding were used to attach the shaped-disc to the collar, this could lead to an undulating surface on the disc which compromises a subsequent weld between the disc and pouch of the ostomy appliance. If an adhesive is used to attach the disc to the pouch, the cost of the ostomy appliance is increased due to the extra raw materials required and through the additional complexity of delivering the correct amount of liquid adhesive to the right place on the shaped-disc and pouch.

US2009/0216208A1 discloses an ostomy carrier device comprising a base plate for securing the carrier device to a user's skin around a stoma; a circumferential flange adapted for connection with and removal of an ostomy collecting bag, the circumferential flange having an opening for receiving a stoma; and at least one connecting ring between the flange and base plate. The connecting ring may be laser welded to the flange and/or base plate.

It is an object of embodiments of the present invention to at least partially overcome or alleviate the above problems.

### Summary of the Invention

In broad terms, the invention concerns a method of manufacturing an ostomy appliance comprising providing at least two layers of material and directing a laser beam onto two of the at least two layers to form a weld between the two layers. The method may be a method of manufacturing a baseplate of an ostomy appliance. One of the at least two layers of material may comprise a collar. One of the at least two layers of material may comprise a shaped-disc. The method may comprise directing the laser onto the shaped disc and collar to form a weld therebetween. The shaped disc may not be substantially flat. The shaped disc may be convex in shape. The shaped disc may be a convex disc.

According to a first aspect of the present invention, there is provided a method of manufacturing a baseplate configured to secure an ostomy appliance to the body of a user, the method comprising providing a convex disc and a collar and directing a laser beam onto the convex disc and collar to form a weld therebetween.

Advantageously, the use of a laser to form a weld between the convex disc and collar allows the weld to be formed quickly and accurately without the need for additional materials or consumables such as an adhesive. In addition, the laser process can be controlled to ensure that the surface of the convex disc remains smooth enough for subsequent manufacturing steps such as attachment to the pouch of an ostomy appliance. In addition, the laser weld provides for adaptation of the weld size and strength without changes in tooling that may be required for other types of welding such as heat and ultrasonic welding.

The laser weld may be configured to provide a fluid-tight seal between the collar and the convex disc. The laser weld may be configured to prevent separation of the convex disc and collar from one another. The convex disc and collar may be secured together without application of an adhesive in a region of overlap between the convex disc and collar. Thus, the laser weld both ensures that gases and liquids cannot pass from the stoma out between the convex disc and collar which prevents unpleasant smells and leaks of liquid. In addition, the laser weld provides the structural integrity of the baseplate by preventing the collar and convex disc from separating from one another without the need for adhesives.

The method may comprise partially melting the convex disc and/or collar during formation of the laser weld. Preferably, both the convex disc and collar are partially melted during formation of the laser weld. The method may comprise melting a melt zone of the convex disc and/or collar. The melt zone may encompass parts of the convex disc and collar. The method may comprise placing a proximal surface of the convex disc in contact with a distal surface of the collar. The method may comprise partially melting the proximal surface of the convex disc. The melt zone may comprise the proximal surface of the convex disc. The method may comprise partially melting the distal surface of the collar. The melt zone may comprise the distal surface of the collar. The method may comprise allowing the convex disc and/or collar to solidify to form the laser weld. Thus, strong and permanent bonds between the convex disc and collar are formed via the laser welding process.

The method may comprise clamping the convex disc and collar together during formation of the laser weld. The method may comprise clamping the convex disc and collar between a support plate and a transfer plate. The support plate and transfer plate may be formed of a material that is more rigid than the convex disc and collar. The support plate and transfer plate may each comprise a clamping surface configured to abut the baseplate during clamping. A, or each, clamping surface may have a shape corresponding to the shape of the convex disc. A, or each, clamping surface may be substantially flat. Preferably, the clamping surface of the transfer plate is flat, especially in regions corresponding to the position of the final laser weld and/or melt zone. A, or each, clamping surface may be substantially smooth. Preferably a clamping surface of the transfer plate is smooth. In this context smooth may mean, for example, that the surface appears smooth to a user, or is free of substantial undulations, protrusions, depressions, or other similar features that might affect its appearance and/or ability to be bound to the pouch. A, or each, clamping surface may be smooth enough to ensure the surface of the convex disc is not substantially altered during welding, preferably even if it melts. Smooth may also preferably mean that the presence of the laser weld is not noticeable to a user during normal use of the pouch. This ensures the convex disc can be easily bound to the pouch after welding and that its aesthetics are not altered by the welding process.

A, or each, clamping surface may comprise a marking provided in relief. The marking may therefore be embossed and/or debossed into the clamping surface. Preferably, the transfer plate comprises a marking provided in relief on a clamping surface of the transfer plate. The method may therefore comprise melting the convex disc during formation of the laser weld such that a corresponding marking is formed in relief on a surface of the convex disc. For example, the melted convex disc may conform to the shape of the marking and then solidify into that shape during the welding process. The marking could be any relevant marking to improve the appearance of the convex disc/baseplate and/or provide information to a user, for example, branding, logos, product identifying information, instructions, or the like. Thus, the laser welding method provides a convenient way to add a marking to the convex disc/baseplate at the same time as it is constructed.

The laser weld may be formed in a part of the convex disc that is visible to a user during normal use. The part of the convex disc containing the laser weld may have a smooth outer surface (distal surface). The marking may be provided in a part of the convex disc that is visible to a user during normal use. The laser weld may not be noticeable during normal use of the convex disc by a user. This can help to ensure any marking applied in the welding process is readable by the user.

The shape of each clamping surface may not change during clamping. The method may comprise maintaining a constant separation between the two clamping surfaces during formation of the weld. The constant separation may be less than a thickness of the baseplate. The method may comprise unclamping the convex disc and collar after formation of the weld, for example after solidification of the convex disc and collar. Thus, the baseplate is supported during formation of the laser weld to ensure a strong and secure weld is formed.

The method may comprise clamping the convex disc and collar together with a clamping force equivalent to a pressure of at least 0.2 Nmm⁻², 0.5 Nmm⁻², 1 Nmm⁻², or 2 Nmm⁻² at the interface between the convex disc and collar. The method may comprise clamping the convex disc and collar together with a clamping force equivalent to a pressure of no more than 2 Nmm⁻², 1 Nmm⁻², 0.5 Nmm⁻², or 0.2 Nmm⁻² at the interface between the convex disc and collar. Preferably, the clamping force is greater than 1 kN, for example 1.2 to 1.6 kN or most preferably about 1.5 kN. Thus the clamping force is selected to ensure a strong weld without damage to the baseplate.

The method may comprise directing the laser onto the convex disc and collar via the transfer plate. The transfer plate may comprise a material that is substantially transparent to the laser. The transfer plate may be formed from glass. Thus, the laser welding process can be performed through the clamping structure and simultaneously with clamping to ensure a strong and secure weld.

The method may comprise directing the laser beam onto the collar via the convex disc. The convex disc may be more transparent to the laser beam than the collar. The transfer plate may abut the convex disc. The transfer plate and/or support plate may comprise non-reflective coating. The non-reflective coating may be applied to the clamping surface. Thus, this arrangement ensures efficient transfer of laser light to form the weld given the convex disc is more transparent than the collar.

The method may comprise directing thermal expansion of the collar and/or convex disc towards the laser weld by clamping the collar and convex disc together. The method may comprise dissipating heat from the convex disc and/or collar during formation of the laser weld. This can help direct thermal expansion as the clamps (transfer plate and support plate) can dissipate heat from the clamped surfaces of the convex disc and collar leading to preferential thermal expansion around the laser weld.

The convex disc may comprise a distal surface opposite the proximal surface of the convex disc. The melt zone may preferably comprise the distal surface of the convex disc. The method may comprise melting substantially the entire thickness of the convex disc during formation of the laser weld. This is preferred to ensure that a stronger and more stable weld is formed. The shape of the convex disc may therefore be controlled by the clamping surface of the transfer plate, for example as mentioned above. This helps maintain the aesthetic appearance of the convex disc, hiding the presence of the weld and also ensuring it is suitable for bonding to the pouch.

In an alternative embodiment, the melt zone may not comprise the distal surface of the convex disc. This may occur where heat dissipation through the transfer plate is high. The melt zone may span at least 10%, 25%, 50%, 70%, 80%, 90%, or 95% of the thickness of the convex disc (i.e. of the distance from the proximal surface to the distal surface of the convex disc).

The method may comprise dissipating heat via the support plate and/or the transfer plate. The method may comprise dissipating heat via the clamping surface of the support plate and/or transfer plate. The support plate may be formed of any suitable material, such as metal or glass. The metal used may be anodised aluminium, anodised steel or stainless steel or another similar metal. Thus, the support plate can be flexibly designed while assisting weld formation.

The collar and/or convex disc may be formed of a thermoplastics material. The thermoplastics material may be a material that volumetrically absorbs the laser beam, preferably to cause melting of the thermoplastics material. Thus, the laser beam may be absorbed through the volume of the thermoplastics material. Volumetric absorption preferably accounts for the majority of, or more preferably substantially all of, the absorption of the laser beam by the thermoplastics material. The method may comprise absorbing part of the laser beam into the convex disc to cause heating, and preferably melting of the convex disc. The method may comprise absorbing part of the laser beam into the collar to cause heating, and preferably melting of the collar. Thus, this ensures that a sufficient amount of laser power reaches the collar to melt it while also imparting a significant amount of energy to the convex disc itself. This causes melting and thermal expansion through the thickness of the convex disc and collar to further strengthen the weld, for example due to increased welding pressure and larger melted volume contributing to the weld.

The laser beam may be selected such that at least 10% of the laser beam is absorbed by the convex disc. The laser beam may be selected such that at least 20% of the laser beam is absorbed by the convex disc. The laser beam may be selected such that at least 30% of the laser beam is absorbed by the convex disc. The laser beam may be selected such that at least 40% of the laser beam is absorbed by the convex disc. The laser beam may be selected such that no more than 50% of the laser beam is absorbed by the convex disc. The laser beam may be selected such that no more than 40% of the laser beam is absorbed by the convex disc. The laser beam may be selected such that no more than 30% of the laser beam is absorbed by the convex disc. The laser beam may be selected such that no more than 20% of the laser beam is absorbed by the convex disc. The laser beam may be selected such that 10-40% of the laser beam is absorbed by the convex disc, more preferably 20-30% of the laser beam may be absorbed by the convex disc. The laser beam may be selected such that the laser beam is volumetrically absorbed by the thermoplastics material to cause melting of the convex disc. This helps ensure sufficient energy is absorbed by both the convex disc and collar. In the following, the term "microns" is interpreted as µm.

The laser used to form the laser weld may have a wavelength outside visible light. The method may comprise using non-visible light to form the weld. The laser may be a microwave laser. The laser may have a wavelength of over 1,000 nm. The laser may have a wavelength of over **1.1** microns, preferably over 1.2 microns. The laser may have a wavelength of over **1.4** microns. The laser may have a wavelength of over 1.6 microns. The laser may have a wavelength of over 1.8 microns. The laser may have a wavelength of over 1.9 microns. The laser may have a wavelength of no more than 3 microns. The laser may have a wavelength of no more than 2.5 microns. The laser may have a wavelength of no more than 2.3 microns. The laser may have a wavelength of no more than 2.2 microns. The laser may have a wavelength of no more than 2.1 microns. The laser may have a wavelength of 1.2 to 2.2 microns. The laser may have a wavelength of 1.6 to 2.2 microns. The laser may have a wavelength of about 2 microns, preferably about 2.0 microns and most preferably 1,940 nm. Thus, the laser wavelength can be selected such that it is easily volumetrically absorbed by the thermoplastics materials to ensure efficient heating through the thickness of the base plate. A wavelength in the range over 1.1 microns is preferred as this is outside the selective heating window of about 0.8 to 1.1 microns. In this selective heating window thermoplastic materials exhibit very low volumetric absorption and require additives in order to absorb sufficient energy to melt. A laser wavelength in the range of 1.2 to 2.2 microns is preferred due to the moderate volumetric absorption properties of thermoplastic materials at these wavelengths. A laser wavelength in the range of 1.6 to 2.2 microns is more preferred as it provides further improved absorption in thermoplastics compared to below 1.6 microns. Around 2 microns and specifically 1940 nm is most preferred due to the wide commercial availability of cheap laser sources at these wavelengths.

The part of the convex disc and/or collar that melts during formation of the laser weld may be free from additives configured to absorb visible light. The melt zone may be free from additives configured to absorb visible light, with no such additives between the convex disc and the collar, or in either the convex disc or the collar. Thus, the laser is selected such that the visible colour of the baseplate does not need to be altered through the addition of additives, such as carbon black, which may make the product unacceptable from a quality / user appeal perspective.

The laser may be a continuous wave laser. The laser power density incident at on the convex disc and collar during welding may be at least 20 Wmm⁻², 40 Wmm⁻², 60 Wmm⁻², or 80 Wmm⁻². The laser power density incident at on the convex disc and collar during welding may be no more than 80 Wmm⁻², 60 Wmm⁻², 40 Wmm⁻², or 20 Wmm⁻². Preferably the laser power density is 60-65 Wmm⁻². Thus, the laser weld can be efficiently formed.

The laser beam may be directed at an angle of close to 90 degrees to the interface between the convex disc and collar. The laser beam may be directed onto the transfer plate at an angle of close to 90 degrees to a top surface of the transfer plate. The top surface may be opposite the clamping surface. The top surface may be substantially parallel to the clamping surface. The top surface may be substantially parallel to the clamping surface in regions corresponding to the final position of the laser weld and/or melt zone. The top surface may be substantially parallel to the interface between the convex disc and clamping surface. The top surface may be substantially parallel to the interface between the convex disc and clamping surface in regions corresponding to the final position of the laser weld and/or melt zone.

The laser may be moved across the convex disc and collar to form the laser weld at a speed of at least 100 mm²s⁻¹, 200 mm²s⁻¹, 400 mm²s⁻¹, 600 mm²s⁻¹, or 800 mm²s⁻¹. The laser may be moved across the convex disc and collar to form the laser weld at a speed of no more than 1000 mm²s⁻¹, 800 mm²s⁻¹, 600 mm²s⁻¹, or 400 mm²s⁻¹. Preferably, the laser forms the weld at a speed of about 600 mm²s⁻¹. Of course, the laser speed may vary significantly in different embodiments of the invention depending on the laser technology employed to form the weld. The laser beam may trace a circular pattern while forming the laser weld. The laser may be directed at the convex disc and collar for a lasing time. The lasing time may be no more than 5 seconds, 4 seconds, 3 seconds, 2 seconds, or 1 second. Preferably, the lasing time is 2 seconds. After the lasing time has elapsed, the laser may not be directed at the convex disc and collar. Thus, the laser weld is formed very quickly.

The method may comprise clamping the convex disc and collar after the lasing time has elapsed. The method may comprise clamping the convex disc and collar for a holding time. The convex disc and collar may cool and/or solidify during the holding time. The holding time may be at least 0.1, 0.25, 0.5, 0.75, or 1 second. The holding time may no more than 2, 1, 0.75 or 0.5 seconds. Thus, the laser weld is given time to form through the curing and solidification of the convex disc and collar.

The method may comprise unclamping the convex disc and collar. Thus, the baseplate is released from the clamp for subsequent attachment to an ostomy appliance or coupling element as described below.

The collar may be configured to secure the baseplate to the body of a user. The collar may comprise a stomal aperture. The laser weld may be formed around a periphery of the stomal aperture. The method may comprise directing the laser to locations around a periphery of the stomal aperture. The stomal aperture be configured to surround the stoma of the user. The stomal aperture may initially be no more than 50, 40, 30, 20 or 10 mm in diameter. The collar may be flexible. The collar may be deformable. Preferably, the stomal aperture is deformable. The stomal aperture may be expandable to a size equivalent to the aperture of the convex disc described below. Thus, the user can efficiently attach the baseplate to their body and shape the stomal aperture such that a comfortable and efficient fit around their stoma is achieved.

The collar may comprise a proximal surface configured to contact the body of a user in use. The proximal surface may be opposite the distal surface of the collar. The proximal surface of the collar may not be melted during formation of the weld. The melt zone may not comprise the proximal surface of the collar. The melt zone may not span the entire thickness of the collar. The proximal surface may comprise an adhesive, and preferably the collar is be formed from an adhesive material. The adhesive material is preferably a mouldable adhesive, for example formed of a hydrocolloid adhesive gel. Alternatively, the method may comprise applying an adhesive to the proximal surface of the collar. Thus, the collar can be efficiently and securely attached to the user of the body via the proximal surface.

The method may comprise deactivating the proximal surface of the collar. The method may comprise providing a release liner on the proximal surface of the collar. The collar may be provided with the release liner. The release liner may be configured to prevent the proximal surface from prematurely attaching to another object. The release liner may be configured to protect the proximal surface. The release liner may be removable from the proximal surface. The release liner may comprise a tab. The tab may be configured to allow the user to grasp the release liner and remove it from the collar. The tab may extend from an outer edge of the release liner, for example in a plane defined by the release liner. The tab may be any suitable shape or size, for example: circular, ring-shaped - for example a pull-ring, elliptical, lens-shaped, semi-circular, triangular, rectangular, square. or irregularly shaped. Preferably, the tab is a semi-circular protrusion. Thus, the proximal surface is protected by the release liner to ensure it is able to strongly adhere to the user when needed and also to prevent inadvertent adhesion to other objects prior to use.

As mentioned above, the collar and convex disc preferably comprise a thermoplastics material. The collar may comprise a thermoplastic material adjacent to the convex disc, for example on the distal surface of the collar. The convex disc may comprise a thermoplastic material adjacent to the collar, for example on the proximal surface of the collar. Preferably, a thermoplastics material forms the majority, or substantially all, of the convex disc. The thermoplastics material may comprise a material that is suitable for laser welding. The thermoplastics material may comprise cyclic olefin copolymer (COC), polycarbonate (PC), poly(methyl methacrylate) (PMMA), acrylonitrile butadiene styrene (ABS), polyethylene (high density and low density), ethylene-vinyl acetate (EVA), ethylene methyl acrylate (EMA) or the like. Thus, the collar and convex disc are able to absorb the laser light and efficiently form a laser weld therebetween.

The convex disc may be no more than 5 mm thick. The convex disc may be no more than 2 mm thick. The convex disc may be no more than 1 mm thick. The convex disc may be at least 0.5 mm thick. The convex disc may be at least 1 mm thick. The convex disc may be at least 1.5 mm thick. Preferably, the convex disc is about 1 mm thick. The convex disc may be the thicknesses mentioned above in the region corresponding to the laser weld. These thicknesses are preferred as they help to ensure an appropriate amount of laser absorption through the convex disc when it is formed of a thermoplastics material, while also ensuring good structural strength.

The distal surface of the collar may not comprise an adhesive. The collar may comprise a backing film forming the distal surface of the collar. The method may comprise laminating the distal surface of the collar with the backing film. The backing film may have a thickness of at least 5 µm, 10 µm, 20 µm, 50 µm, 100 µm, or 200 µm. The backing film may have a thickness of no more than 200 µm, 100 µm, 50 µm, 30 µm, 20 µm, or 10 µm. Preferably the backing film has a thickness of about 100µm or in another embodiment 15µm. The melt zone may comprise the backing film. The melt zone may encompass at least 10%, 25%, 50%, 75% or 90% of a thickness of the backing film. The melt zone of the collar may encompass only the backing film (and no other parts of the collar). The melt zone preferably encompasses over 50% of the thickness of the backing film, most preferably the melt zone encompasses the entire thickness of the backing film. The backing film may be formed of a thermoplastics material as described above.. The backing film may not comprise an adhesive. The adhesive material of the collar may be melted during welding. The adhesive material may be melted due to thermal conduction of heat from the backing film and/or absorption of the laser beam. The method may comprise attaching a backing film to the collar to form the distal surface, for example by laminating the backing film onto the collar. The backing film may be configured to prevent adhesion of the collar/distal surface to other objects. The backing film may be configured to protect the adhesive material of the collar. Thus, the collar is protected by the backing film.

The collar may be substantially annular. The collar may be sheet-like. The collar may be substantially flat. The collar may be flat until it is attached to the convex disc. The convex disc may at least partially define the shape of the collar. The method may comprise conforming the shape of the collar to the shape of the convex disc. The collar may be shaped prior to attachment to the convex disc, for example by thermoforming. Shaping the collar can help ensure it fits onto the convex disc more easily and without damage to the collar - for example due to excessive deformation when conforming to the shape of the convex disc. The collar may have an inner perimeter defined by the stomal aperture. The collar may have an outer perimeter. The outer perimeter may define an outer edge of the base plate. The collar may have an outer width of at least 100, 120, 140 or 160 mm.

The convex disc may be configured to secure the baseplate to a pouch of an ostomy appliance. The baseplate may be configured to bear against the abdomen of the user, preferably to provide better access to the user's stoma. The convex disc may provide the shape of the baseplate. The convex disc may be formed from a material that is more rigid than the collar. The method may comprise conforming the collar to the shape of the convex disc. The convex disc may be formed from a thermoplastics material. The convex disc may be formed from a hard plastics material, for example a thermoplastic material like cyclic olefin copolymer (COC), polycarbonate (PC), poly(methyl methacrylate) (PMMA), acrylonitrile butadiene styrene (ABS), high-density polyethylene (HDPE) or alternatively low density polyethylene (LDPE) or ethylene vinyl-acetate (EVA) as described above. The convex disc may be formed of a material configured to absorb the light from the laser. Thus, the convex disc enables the baseplate to be securely attached to a variety of stomas including recessed stomas.

The convex disc may be annular. The convex disc may be sheet-like. The convex disc may comprise an aperture. The aperture may be located centrally located in the convex disc. The aperture may be at least as big as the stoma of a user. Preferably the aperture is larger than a typical stoma of a user so that it may easily fit around the stoma in use. The aperture may be no more than 100, 90, 80, 70, or 60 mm in diameter The aperture may be at least 10, 20, 30, 40, 50, 60, 70, 80 or 90 mm in diameter, for example it may be 70 mm in diameter. The aperture may be larger than the stomal aperture. The laser weld may be formed around a periphery of the aperture. The method may comprise directing the laser to locations around a periphery of the aperture. Thus, the convex disc fits around the stoma and the laser weld is formed around this to provide a fluid-tight seal.

The convex disc may comprise a flat region. The flat region may be substantially planar. The flat region may be annular. The method may comprise directing the laser onto the flat region to form the laser weld. The method may comprise forming the laser weld in the flat region. The flat region may comprise the melt zone. The melt zone may be completely contained within the flat region. The flat region may form an outer region of the convex disc. The flat region may form an outer edge of the convex disc. Thus, the laser weld can be better formed due to the flat nature of the flat region.

The convex disc may comprise a shaped region. The shaped region may not be flat. The shaped region may be arranged concentrically within the flat region. An outer perimeter of the shaped region may join to an inner perimeter of the flat region, and preferably both respective perimeters are the same. The shaped region may be sheet-like. The method may not comprise directing the laser onto the shaped region. The shaped region may not comprise the melt zone. The shaped region may form an inner region of the convex disc. The inner perimeter of the shaped region may form the aperture of the convex disc. The shaped region may extend out of a plane defined by the flat region. The shaped region may extend in a direction that is towards the user in use. Thus, the shaped region helps to expose the stoma for tight engagement between the collar and user's body. In addition. the weld is not formed in the shaped region is not flat and therefore not ideal for weld formation.

The convex disc/flat region may have an outer width of at least 50, 70, 80, 90, 100 or 120 mm. The convex disc/flat region may have an outer width of no more than 120, 100, 90, or 80 mm. The flat region may have an inner width of at least 40, 50, 60 70, or 80 mm. The flat region may have an inner width of no more than 80, 70, 60, or 50 mm. The convex disc may have an outer width that is smaller than the outer width of the collar. The outer perimeter of the collar may extend outside the outer perimeter of the convex disc, preferably in all directions. Thus, the user may directly press part of the collar into the body such that it can form a tight adhesive bond with the skin.

The laser weld may be any suitable shape or size, but is preferably a continuous loop. The laser weld may have an inner perimeter. The laser weld may have an outer perimeter. The inner and outer perimeter may be the same shape. The inner and/or outer perimeter may be the same shape as the flat region and/or shaped region. The laser weld may be annular, for example with an inner diameter and an outer diameter. The laser weld may comprise an inner width that is at least 50, 60, 70, 80 or 90 mm. The laser weld may comprise an inner width that is no more than 90, 80, 70, 60 or 50 mm. The laser weld may comprise an outer width that is at least 0.5, 1, 2, 5, 10 or 15 mm greater than its inner width. The laser weld may comprise an outer width that is no more than 20, 15, 10, 5, 2 or 1 mm greater than its inner width. Preferably, the outer width of the laser weld is 2 mm greater than the inner width of the laser weld. Thus, the laser weld provides a secure attachment between the collar and convex disc.

As described above, the convex disc may comprise a proximal surface and an opposite distal surface. The collar may comprise a proximal surface and an opposite distal surface. The proximal surface of the collar may contact the body of a user in use. The proximal surface of the collar preferably comprises an adhesive. The release liner may be provided over the proximal surface of the collar. The method may comprise placing the proximal surface of the convex disc in contact with the distal surface of the collar. The distal surface of the collar may comprise a backing film. The method may comprise forming a laser weld between the proximal surface of the convex disc and the distal surface of the collar, and preferably between the proximal surface of the convex disc and the backing film.

The method may comprise forming two or more laser welds between the convex disc and collar. Each of the two or more laser welds may have any one or more of the optional features of the weld mentioned above and may be formed according to the method described above and any one or more of the optional features of that method. Each of the two or more laser welds may be different or may be formed differently. Each of the two or more laser welds may be independent. The method may comprise forming two concentric laser welds. Thus, additional functionality can be provided by providing two or more laser welds.

The method may comprise forming a load-bearing weld. The load-bearing weld may be configured to prevent separation of the two layers from one another. The two or more laser welds may comprise the load-bearing weld.

The method may comprise forming a sealing weld. The sealing weld may be configured to provide a fluid-tight seal between the convex disc and collar. The sealing weld may be formed around the inner periphery of the convex disc. The two or more laser welds may comprise the load-bearing weld.

The method may comprise forming a load-bearing weld and a sealing weld. The load-bearing weld may be formed outside the sealing weld, for example concentrically outside. Thus, good sealing and attachment properties can be provided by two dedicated laser welds.

In a preferred embodiment, there is provided a method of manufacturing a baseplate configured to secure an ostomy appliance to the body of a user, the method comprising providing a convex disc and a collar and directing a laser beam onto the convex disc and collar to form a laser weld therebetween, wherein the method includes absorbing part of the laser beam into the convex disc to cause melting of the convex disc. Preferably, the laser beam is absorbed volumetrically by the convex disc.

In a preferred embodiment, there is provided a method of manufacturing a baseplate configured to secure an ostomy appliance to the body of a user, the method comprising providing a convex disc and a collar and directing a laser beam onto the convex disc and collar to form a laser weld therebetween wherein the laser beam has a wavelength of 1.2 to 2.2 microns, preferably, 1.6 to 2.2 microns and most preferably about 2 microns.

In a preferred embodiment, there is provided a method of manufacturing a baseplate configured to secure an ostomy appliance to the body of a user, the method comprising providing a convex disc and a collar, directing a laser beam onto the convex disc and collar to form a laser weld therebetween, and clamping the convex disc and collar together during formation of the laser weld between a support plate and a transfer plate, wherein the transfer plate comprises a marking provided in relief on a clamping surface of the transfer plate and the method comprises clamping the transfer plate against the convex disc and melting the convex disc during formation of the laser weld such that a corresponding marking is formed in relief on a surface of the convex disc.

In a preferred embodiment, there is provided a method of manufacturing a baseplate configured to secure an ostomy appliance to the body of a user, the method comprising providing a convex disc and a collar and directing a laser beam onto the convex disc and collar to form a laser weld therebetween, wherein the method comprises either: directing the laser beam onto the collar via the convex disc and melting substantially the entire thickness of the convex disc during formation of the laser weld. Preferably, the collar comprises a backing film and an adhesive material, and the method comprises melting substantially the entire thickness of the backing film during formation of the laser weld.

The baseplate may comprise a secondary body attachment. The convex disc may comprise the secondary body attachment. The secondary body attachment may be configured to secure the baseplate to the body of a user. The secondary body attachment may comprise a pair of belt mounts. The belt mounts may be positioned on opposite sides of the baseplate. The belt mounts may extend from the outer perimeter of the convex disc. Thus, the secondary body attachment helps ensure the ostomy appliance doesn't move during use and provides additional security to the user in the event the adhesive of the collar fails.

According to a second aspect of the present invention there is provided a method of manufacturing a body fitment component for a two-piece ostomy appliance, the method comprising manufacturing a baseplate according to the first aspect of the invention, wherein the convex disc is provided with a coupling element for coupling to a corresponding coupling element on a pouch.

The method of the second aspect may include any optional feature of the first aspect as outlined above.

The coupling elements may be of the snap-on type. One or both coupling elements may be resiliently deformable to snap into engagement with one another. Thus, the coupling elements can be easily but securely fastened to one another.

Preferably, the laser weld is formed before attaching the baseplate to the coupling element. This makes manufacturing easier and is possible as the laser weld does not cause changes in the surface properties of the convex disc that could render it unsuitable for attachment to the coupling element.

A coupling weld may be formed between the coupling element and convex disc. The coupling weld may be formed between the coupling element and the distal surface of the convex disc. The coupling weld may be formed after formation of the laser weld. The method may comprise forming the coupling weld between the coupling element and the baseplate via any suitable means such as: weld; mechanical seal; heat seal; pressure seal; adhesive; solvent bond; ultraviolet bond; ultrasonic weld; laser weld; impulse weld; or friction weld. Preferably, the coupling weld is a heat weld formed by heat sealing. Thus, the coupling weld allows the baseplate to be attached to the coupling element and pouch.

The coupling weld may overlap with the laser weld. The coupling weld may be the same shape as the laser weld. The coupling weld may have an outer perimeter (e.g. diameter) that extends outside an inner perimeter (e.g. diameter) of the laser weld. The coupling weld may have an inner perimeter (e.g. diameter) than extends within an outer perimeter (e.g. diameter) of the laser weld. The coupling weld may have an outer perimeter (e.g. diameter) that extends inside an outer perimeter (e.g. diameter) of the laser weld. The coupling weld may have an outer perimeter (e.g. diameter) that extends inside a marking provided in relief on the convex disc. Thus, the available space can be maximised due to the laser weld leaving the convex disc in a suitable flat state for subsequent welding, such as heat sealing. This ensures both welds are strong and secure and also leaves part of the laser weld visible to the user such that any markings formed on the convex disc in the laser welding process are readable.

According to a third aspect of the present invention there is provided a method of manufacturing an ostomy appliance, the method comprising providing a pouch, and either manufacturing a baseplate according to the first aspect of the invention and attaching the baseplate to the pouch, or manufacturing a body fitment component according to the second aspect of the invention and providing the corresponding coupling element on the pouch.

The method of the third aspect may include any optional feature of the first and second aspects as outlined above.

Preferably, the laser weld is formed before attaching the baseplate to the pouch. This makes manufacturing easier and is possible as the laser weld does not cause changes in the surface properties of the convex disc that could render it unsuitable for attachment to the pouch.

The method may comprise attaching the body fitment component to the pouch via the coupling elements.

A coupling weld may be formed between the pouch and convex disc. The coupling weld may be formed between the pouch and the distal surface of the convex disc. The coupling weld may be formed after formation of the laser weld. The method may comprise forming the coupling weld between the pouch and the baseplate via any suitable means such as: weld; mechanical seal; heat seal; pressure seal; adhesive; solvent bond; ultraviolet bond; ultrasonic weld; laser weld; impulse weld; or friction weld. Preferably, the coupling weld is a heat weld formed by heat sealing. Thus, the coupling weld allows the baseplate to be attached to the pouch.

The coupling weld may overlap with the laser weld. The coupling weld may be the same shape as the laser weld. The coupling weld may have an outer perimeter (e.g. diameter) that extends outside an inner perimeter (e.g. diameter) of the laser weld. The coupling weld may have an inner perimeter (e.g. diameter) than extends within an outer perimeter (e.g. diameter) of the laser weld. Thus, the available space can be maximised due to the laser weld leaving the convex disc in a suitable flat state for subsequent welding, such as heat sealing. This ensures both welds are strong and secure.

According to a fourth aspect of the present invention there is provided a baseplate for securing an ostomy appliance to the body of a user comprising a convex disc and a collar, and a laser weld between the convex disc and collar.

The baseplate may be manufactured according to the first aspect of the invention and may include any optional feature of the first aspect as described above.

According to a fifth aspect of the present invention there is provided an ostomy appliance comprising the baseplate of the fourth aspect of the invention and a pouch.

The ostomy appliance of the fifth aspect may be manufactured according to the second or third aspects of the invention and may include any optional feature of the second or third aspects as described above.

According to a sixth aspect of the present invention there is provided a method of using an ostomy appliance according to the fifth aspect of the present invention, the method comprising attaching the baseplate to the body of the user around a stoma and allowing stomal effluent to pass from the stoma and into the pouch via the base plate.

### Detailed Description of the Invention

In order that the invention may be more clearly understood one or more embodiments thereof will now be described, by way of example only, with reference to the accompanying drawings, of which:
- Figure 1: is a schematic perspective view of a first embodiment of an ostomy baseplate;
- Figure 2: is a schematic plan view of the ostomy baseplate of Figure 1 with the position of the laser weld and heat weld shown;
- Figure 3: is a schematic perspective view of the convex disc of the ostomy baseplate of Figure 1;
- Figure 4: is a schematic plan view of a collar of the ostomy baseplate of Figure 1;
- Figure 5: is a schematic exploded view of the ostomy baseplate of Figure 1;
- Figure 6: is a schematic cross-sectional view of an ostomy appliance comprising the ostomy baseplate of Figure 1;
- Figure 7: is a flow diagram of the method of forming the ostomy baseplate of Figure 1;
- Figure 8: is a schematic cross-sectional view of the ostomy baseplate of Figure 1 before formation of the laser weld;
- Figure 9: is a schematic cross-sectional view of the ostomy baseplate of Figure 1 during formation of the laser weld;
- Figure 10: is a schematic cross-sectional view of the ostomy baseplate of Figure 1 during formation of the laser weld;
- Figure 11: is a schematic cross-sectional view of the ostomy baseplate of Figure 1 after formation of the laser weld;
- Figure 12: is a schematic plan view of a second embodiment of an ostomy baseplate;
- Figure 13: is a schematic cross-sectional view of a third embodiment of an ostomy baseplate before formation of the laser weld;
- Figure 14: is a schematic cross-sectional view of the ostomy baseplate of Figure 13 during formation of the laser weld;
- Figure 15: is a schematic cross-sectional view of the ostomy baseplate of Figure 13 during formation of the laser weld; and
- Figure 16: is a schematic cross-sectional view of the ostomy baseplate of Figure 13 after formation of the laser weld.

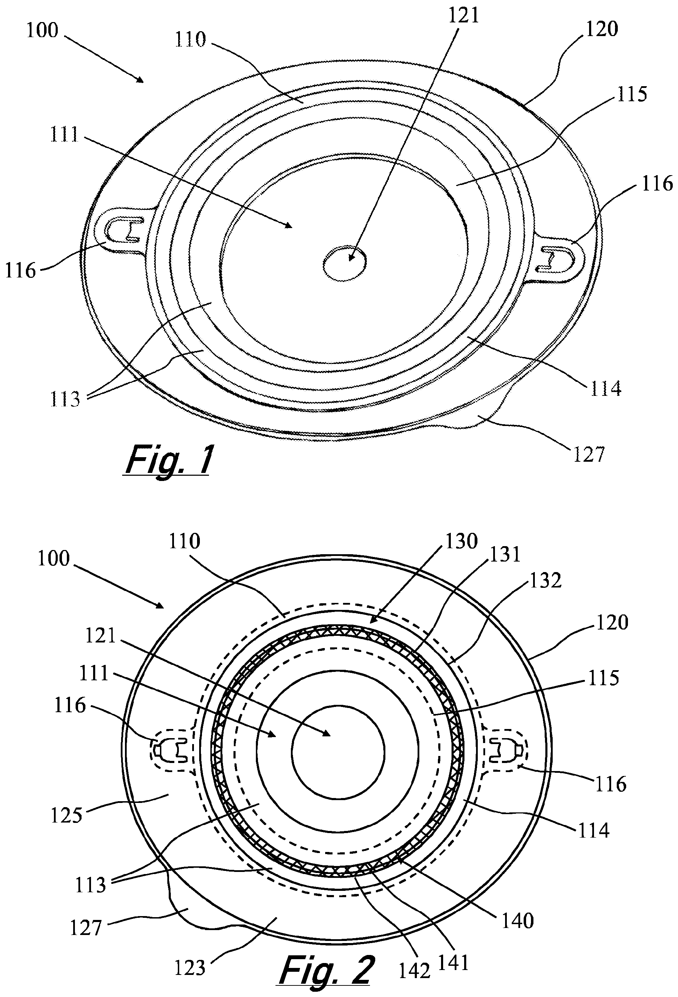

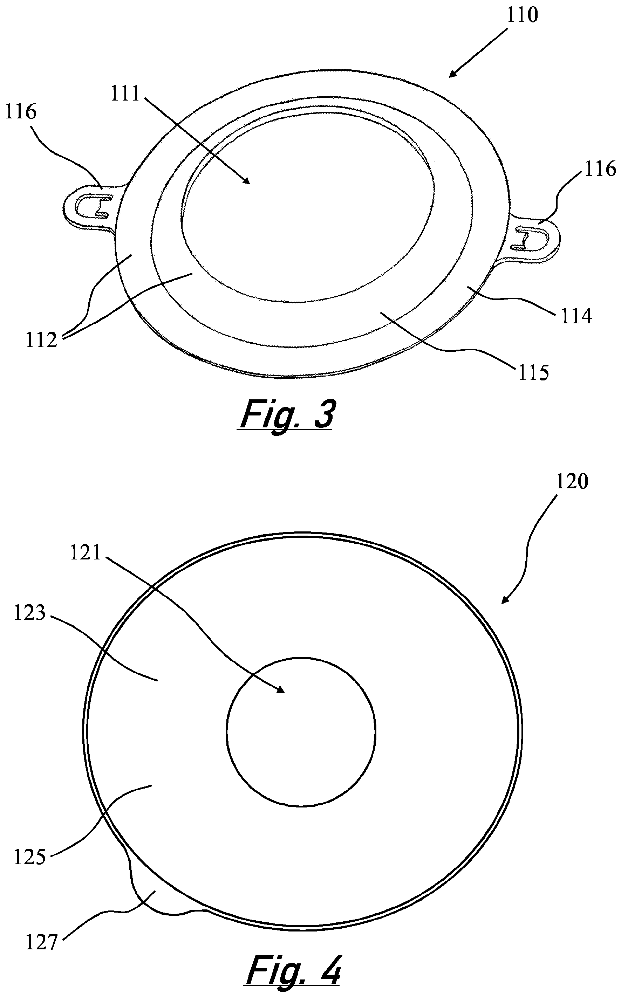

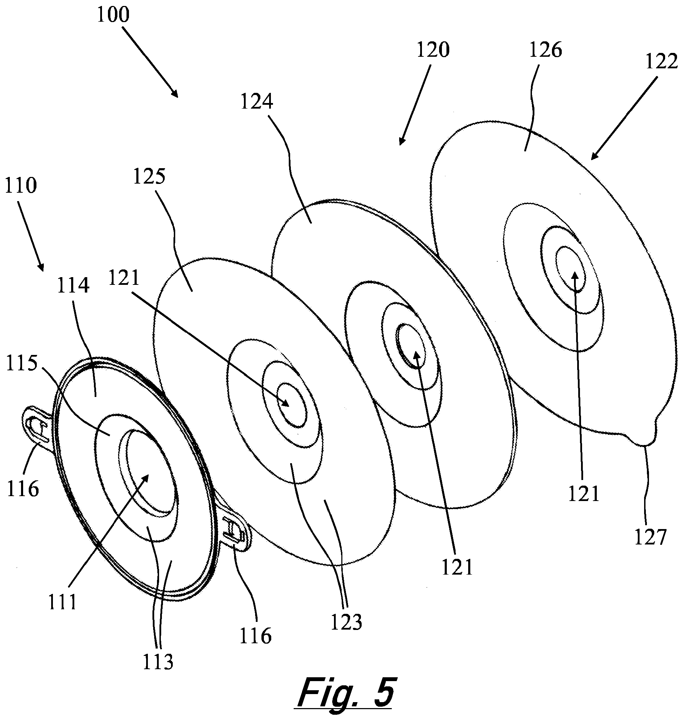

Referring to Figures 1-5, a first embodiment of an ostomy baseplate 100 comprises a shaped-disc in the form of a convex disc 110 and a collar 120.

In this embodiment, the convex disc 110 is generally annular in shape with a sheet-like profile. The convex disc 110 comprises an aperture 111 located centrally in the convex disc 110 and sized to fit around the stoma of a user. The convex disc 110 comprises a proximal surface 112 intended to be facing the abdomen of the user in use, and an opposite distal surface 113.

In this embodiment, the convex disc 110 comprises a flat region in the form of an outer region 114, and a shaped region that is the inner region 115 concentrically within the outer region 114 and forming the edge of the aperture 111. The inner region 115 extends out of the plane of the disc 110 in a direction that is towards the user in use, thus the profile of the convex disc 110 helps the baseplate 100 to press the abdomen of the user into their body around the stoma. This acts to expose a recessed stoma and facilitate a better fluid connection to the stoma. It can also be helpful where the user is overweight and/or has a stoma with complications such as scarring.

In this embodiment, the proximal surface 112 of the convex disc 110 is flat at points corresponding to the outer region 114 and has convex curvature across the inner region 115 before it meets the aperture 111. Correspondingly, the distal surface 113 is also flat at points corresponding to the outer region 114 but has concave curvature across the inner region 115. Advantageously, the convex curvature of the proximal surface 112 ensures that the part of the baseplate 100 pressing into the abdomen is smoothly curved to provide an even application of pressure over a larger surface area to enhance user comfort and reduce the likelihood of damage to the collar 120.

In this embodiment, the convex disc 110 further comprises a secondary body attachment in the form of pair of belt mounts 116 diametrically opposed from one another about the outer edge of the convex disc 1120. The belt mounts 116 are configured to allow attachment of a belt (not shown) to provide additional support to the baseplate 100/ostomy appliance when attached to the body of a user. This helps ensure the ostomy appliance doesn't move during use and provides additional security to the user in the event the adhesive of the collar fails. The exact configuration of the belt mounts 116 is not critical and in different embodiments a variety of different belt mounts 116 could be used, or they could be omitted entirely and replaced with a different type of body attachment. In this embodiment, the belt mounts 116 are those as described in WO2021/123786A1.

In this embodiment, the convex disc 110 is a circular annulus and has an outer diameter of about 90 mm, for example 87 mm, and an inner diameter defining the aperture 111 of 25-65 mm, for example 45 mm. The outer region 114 has an inner and outer diameter of 61 and 87 mm respectively and the inner region 115 therefore has an inner and outer diameter of 45 and 61 mm respectively. Of course, these shapes and dimensions are purely exemplary and other shapes and dimensions may be more suited to other embodiments of the invention.

In this embodiment, the proximal surface 112 on the outer region 114 is angled with respect to the proximal surface 112 on the inner region 115 at the point the outer and inner regions 114, 115 meet. In this embodiment, it is angled by 70 degrees but in other embodiments, the angle between the inner and outer regions may be different, for example 30-90 degrees, or 40-80 degrees as appropriate. The inner region 115 then curves such that the proximal surface 112 adjacent the aperture 111 is parallel to the proximal surface 112 on the outer region 114.

In this embodiment, the plane of the aperture 111 is parallel to but offset from the plane of the outer region 114 by 7 mm. This ensures that a recessed stoma is effectively exposed by the baseplate 100 during use. Of course, in other embodiments different offsets can be used to achieve the same effect and the offset may be tailored to the user to achieve optimal results.

In this embodiment, the convex disc 110 is generally the most rigid part of the ostomy baseplate 100 so that it can bear against the abdomen of the user during use to expose the stoma. Consequently, the convex disc 110 is formed from a hard plastics material. A thermoplastic material is preferred such as cyclic olefin copolymer (COC), polycarbonate (PC), poly(methyl methacrylate) (PMMA), acrylonitrile butadiene styrene (ABS), high-density polyethylene (HDPE), ethylene methyl acrylate (EMA) or in this embodiment ethylene-vinyl acetate (EVA). This allows the convex disc 110 to expand when heated during melding which increases welding pressure resulting in a stronger weld and is also able to volumetrically absorb the laser beam as mentioned below.

In this embodiment, the collar 120 is also annular in shape with an outer diameter at least as large as the outer diameter of the convex disc 110, for example in this embodiment it is 120 mm. The inner diameter of the collar 120 is no larger than the inner diameter of the convex disc 110, for example in this embodiment the inner diameter of the collar 120 is 20 mm. Consequently, the collar 120 comprises a stomal aperture 121 defined by its inner diameter that is preferably smaller than the aperture 111 in the convex disc 110 and which, in use, is aligned with aperture 111 on the same axis.

In this embodiment, as with the convex disc 110, the collar 120 comprises a proximal surface 122 intended to be facing the abdomen of the user in use, and an opposite distal surface 123.

In this embodiment, the collar 120 comprises an adhesive, namely a mouldable adhesive 124. The adhesive 124 defines the inner and outer diameters of the collar 120 and thus comprises an annular disc of adhesive material, for example a hydrocolloid adhesive or other suitable adhesive that has a high propensity to adhere to skin and is mouldable to adjust to the shape and size of the stoma.

In this embodiment, the mouldable adhesive 124 is laminated with a backing film 125 on the side corresponding to the distal surface 123 of the collar 120. The backing film 125 is configured to prevent the mouldable adhesive 124 from damage as well as unintentional adhesion of the adhesive 124 to other objects via the distal surface 123 of the collar 120. The backing film 125 also prevents liquid ingress into the mouldable adhesive 124, such as during washing or bathing. As such, the backing film 125 is laminated over the entire area of the mouldable adhesive 124 on the side corresponding to the distal surface 123 of the collar 120. Consequently in this embodiment the backing film 125 has a thickness of about 100 microns.

In this embodiment, a laser weld 130 is present between the backing film 125 and convex disc 110 as described below and as such, the backing film 125 is formed from a material that is suitable for laser welding using the chosen laser wavelength. For example when lasing at a wavelength of over 1.1 microns a suitable thermoplastics material can volumetrically absorb the laser radiation directly, causing heating and melting once a sufficient amount of energy is absorbed. Such a thermoplastics material could be polyethylene as used in this embodiment, or the thermoplastics materials mentioned above in relation to the convex disc. In this embodiment, a laser wavelength of 1,940 nm is preferred due to good volumetric absorption by thermoplastics and wide availability of cheap 1,940 nm laser sources.

In this embodiment, the collar 120 comprises a release liner 126 configured to cover the entire mouldable adhesive 124 on a side corresponding to the proximal surface 122 of the collar 120. The release liner 126 is configured to prevent inadvertent adhesion of the adhesive 124 to other objects via the proximal surface 123 of the collar 120. The release liner 126 is configured to be removed from the collar 120 prior to use such that the adhesive 124 may be used to attach the baseplate 100 to the user as described below.

In this embodiment, the release liner 126 comprises a tab 127 which is a semi-circular protrusion extending from the outer edge of the release liner 126 and in the plane of the release liner 126. The tab 127 allows the user to more easily grasp the release liner 126 and separate it from the adhesive 124 as the tab 127 extends out past the outer diameter of the collar 120.

Of course, in other embodiments, the backing film, release liner and/or tab may be omitted entirely or have different properties from those described here, for example they may be formed from different materials or have different sizes and shapes depending on the specific embodiment. In addition, the collar itself may have a different physical shape/size depending on the specific embodiment.

Referring to Figure 2, in this embodiment the convex disc 110 is attached to the collar 120 by a laser weld 130 that is present between the convex disc 110 and backing film 125. The laser weld 130 thereby attaches the proximal surface 112 of the convex disc 110 to the distal surface 123 of the collar 120. The laser weld 130 is annular and arranged in a position corresponding to the flat region 114 of the convex disc 110. The laser weld 130 is defined by an inner diameter 131 and an outer diameter 132 centred on the axis of the baseplate 100 and thereby provides an annular and irreversible weld around the stoma that is fluid-tight and able to ensure the convex disc 110 and collar 120 do not become separated during use. In this embodiment, the laser weld has an inner diameter 131 of 75-85 mm, for example 81 mm, and an outer diameter 132 that is larger than the inner diameter 131 and also of 75-85 mm, for example 83 mm. Consequently, the laser weld 130 therefore covers an area of about 250 mm².

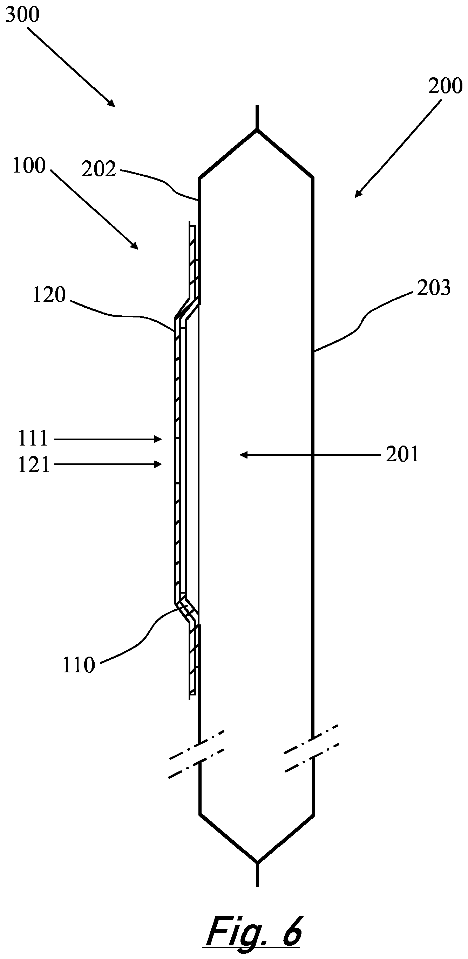

Referring to Figures 2 and 6, in this embodiment an ostomy appliance 300 comprises the ostomy baseplate 100 attached to a pouch 200. The pouch 200 is formed from a rear wall 202 and a front wall 203 joined about their peripheries to form an enclosed space for collecting stomal output. The rear wall 202 comprises a stomal inlet 201 which is a hole in the rear wall 202 that corresponds with the position of the aperture 111 of the convex disc 110 and stomal aperture 121 of the collar 120 and is configured to allow stomal output to pass from the stoma into the pouch 200.

In this embodiment, the baseplate 100 is attached to the pouch 200 via a coupling weld in the form of a heat weld 140 (see fig 2) present between the convex disc 110 and rear wall 202. As with the laser weld 130, the heat weld 140 is annular and is present in the flat region 114 of the convex disc 110. The heat weld 140 is defined by an inner diameter 141 and an outer diameter 142.

In this embodiment, the heat weld 140 has an inner diameter 141 of 65-75 mm for example 69 mm and an outer diameter 142 of 70-80 mm, for example 75 mm. The outer diameter 142 of the heat weld 140 is therefore larger than the inner diameter 131 of the laser weld 130 and as such, the two welds 130, 140 overlap. This is advantageous as it allows the surface area of each weld to be maximised increasing the overall strength and performance of the bonds formed by each weld. This is only possible because the laser weld 130 can be formed without causing significant changes in the profile of the distal surface 113 of the convex disc 110. In contrast, other welding techniques such as heat or ultrasonic welding would change the surface profile of the distal surface 113 to introduce undulations that render it unsuitable for subsequent heat welding to the pouch 200.

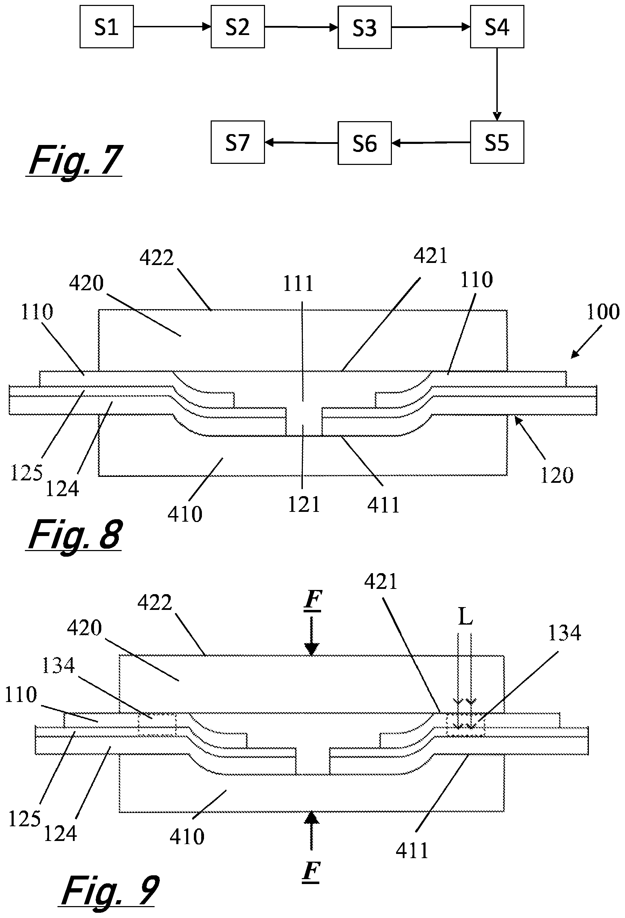

Referring to Figures 7-11, in this embodiment the ostomy appliance 300 is manufactured according to steps S1-S7. This involves first manufacturing a baseplate 100 according to steps S1-S5 by clamping the convex disc 110 and collar 120 between a support plate 410 and a transfer plate 420 and forming a laser weld 130 between the convex disc 110 and collar 120 as described below. The support plate 410 and transfer plate 420 may also be known as a lower clamp and an upper clamp in the art.

In this embodiment, at step S1, the convex disc 110 and collar 120 are aligned co-axially on a clamping surface 411 of the support plate 410 with the proximal surface 122 of the collar 120 in contact with the clamping surface 411. In this embodiment, the release liner 126 is pre-attached to the collar 120 and consequently prevents adhesion of the collar 120 to the clamping surface. The transport plate 420 is then placed over the convex disc 110 and a clamping surface 421 of the transport plate 420 contacts the distal surface 113 of the convex disc 110, for example as shown in Figure 8. This conforms the shape of the collar 120 to the convex disc 110 and ensures they are ready to be welded together.

In this embodiment, the support plate 410 is formed of metal, for example anodized aluminium, stainless steel, anodised steel or another similar metal. The transfer plate 420 is formed of glass and consequently both the support plate 410 and transfer plate 420 are significantly more rigid than both the convex disc 110 and collar 120.

The clamping surface 411 of the support plate 410 is shaped to correspond to the shape of the convex disc 110 to allow it to provide good structural support to the baseplate 100 during welding. The clamping surface 421 of the transfer plate 420 is flat so that there is a tight fit between the flat outer region 114 of the convex disc 110 and transfer plate 420 is achieved such that there are no air gaps at the interface between them.

In this embodiment, the process then moves to step S2 where a clamping force ***F*** is applied to clamp the support plate 410 and transfer plate 420 together with the baseplate 100 therebetween. In this embodiment, a clamping force of 1500 N is used to achieve a clamping pressure of 0.5-2.0 Nmm⁻² at the interface between the convex disc 110 and collar 120. The clamping pressure causes the baseplate 100 to compress slightly due to the flexible resilient nature of the materials used to make the baseplate 100. Importantly, no permanent structural damage or significant changes to the shape of the baseplate 100 result from the applied clamping force ***F.*** Of course, the clamping force ***F*** may vary significantly depending on the materials used to construct the baseplate 100 and needs to be selected to ensure that a strong laser weld is formed without structural damage or excessive deformation of the baseplate 100.

In this embodiment, the process then moves to step S3 where a laser beam L is directed onto the baseplate 100 to form the laser weld 130 while the clamping force ***F*** is maintained. The laser beam L is incident at an incidence angle of close to 90 degrees to a top surface 422 of the glass transfer plate 420 that is opposite from the clamping surface 421. The top surface 422 is parallel to the proximal surface 112 of the convex disc 110 and distal surface 123 of the collar 120. Preferably, the top surface 422 comprises an anti-reflective coating (not shown) which, along with the incidence angle, helps minimise reflections and maximise transfer of the laser beam L into the transfer plate 420. The laser beam L passes through the transfer plate 420, into the convex disc 110 and on into the collar 120.

In this embodiment, 20-30% of the laser beam L is volumetrically absorbed by the convex disc 110 and part of the laser beam is volumetrically absorbed by the backing film 125 of the collar. This causes the convex disc 110 and backing film 125 to be each directly heated by the laser beam L until both the convex disc 110 and backing film 125 melt. In this embodiment, the entire thickness of both the convex disc 110 and backing film 125 melt which is preferred as it leads to a stronger weld through increased thermal expansion of the materials increasing welding pressure and a greater volume of material contributing to the weld..

Heat in the convex disc 110 is simultaneously dissipated by the transfer plate 420 and consequently, the distal surface 113 of the convex disc 110 in contact with the transfer plate 420 does not heat to the same extent. This helps direct thermal expansion preferentially towards the interface of the convex disc and collar.

A melt zone 134 can be defined in the baseplate 110 that represents the volume of the baseplate 100 that is melted during formation of the laser weld 130.In this embodiment, the melt zone 134 is annular with an inner and outer diameter equal to the inner and outer diameter of the laser weld described above. The melt zone 134 extends from the proximal surface 112 of the convex disc 110 to the distal surface 113 of the convex disc 110. The melt zone 134 also extends from the distal surface 123 of the collar 120 all the way through the backing film 125 to the mouldable adhesive 124, for example as shown in Figure 9. In some embodiments, the mouldable adhesive 124 is also melted during welding, for example due to thermal conduction of heat from the backing film 125.

In this embodiment, the laser beam L has a power density of 60-65 Wmm⁻² at a wavelength of 2,000 nm with a beam width of about 1.5-3 mm. The laser beam L traces the entire annular area of the laser weld 130 over a lasing time of 2 seconds that defines the duration of step S3.

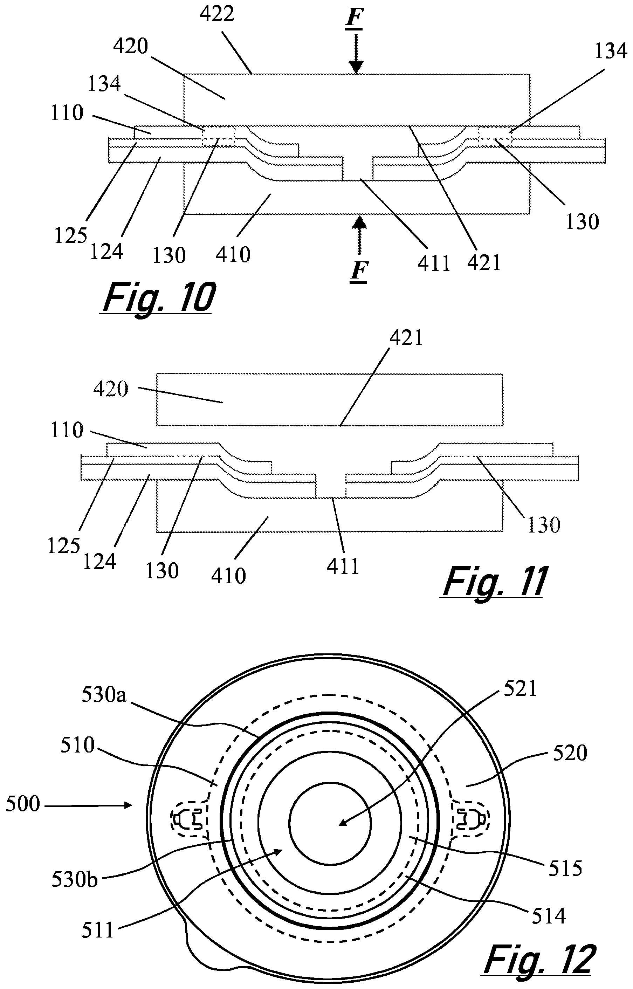

In this embodiment at step S4 the laser beam L is switched off and the clamping force ***F*** maintained for a holding time of 0.25 to 1 seconds, for example 0.5 seconds, which allows the material in the melt zone 134 to cool and begin to solidify. As the solidification progresses the laser weld 130 begins to form between the convex disc 110 and collar 120/backing film 125, for example as shown in Figure 10.

As the whole thickness of the convex disc 110 is melted, the clamping surface 421 of the transfer plate 420 defines the final shape of the convex disc 110 in the region of the weld. In this embodiment, the clamping surface 421 is smooth and flat, advantageously this means the laser weld is not noticeable after formation. This improves subsequent attachment of the baseplate 100 to other objects (see below) and makes it more aesthetically pleasing.

In this embodiment the process then moves to step S5 where the clamping force ***F*** is released and the transfer plate 420 moved away from the support plate 410, for example as shown in Figure 11. Following step S5, the baseplate 100 is fully formed and ready to be combined with a pouch 200 to form the ostomy appliance as described below.

In this embodiment the process then moves to step S6 where a coupling weld in the form of a heat weld 140 is formed between the baseplate 100 and a pouch 200 to form the ostomy appliance 300. In an alternative embodiment, in this step a coupling weld may be formed between the baseplate 100 and a coupling element. A corresponding coupling element may be provided on the pouch 200 for attachment of the baseplate 100 to the pouch 200 via the coupling elements.

In this embodiment, the process then moves to step S7 where the ostomy appliance 300 is ready for use by the user.

In this embodiment, the release liner (not shown in Figures 8-11) is provided on the collar 120 before the laser weld is formed, but in other embodiments the release liner may be provided after the laser weld is formed as required. In addition, the aperture 121 in the collar 120 is provided before the laser weld 130 is formed, but in other embodiments, the aperture 121 may be cut out of the collar 120 after the laser welding process.

Referring to Figure 12, a second embodiment of an ostomy baseplate 500 comprises many of the same features as the first embodiment described above and as such only the differences between the two embodiments are described. In addition, like numerals are used to denote like features.

In this embodiment, the baseplate 500 comprises two laser welds 530a, 530b between the convex disc 510 and collar 520: a load-bearing weld 530a and a sealing weld 530b. Both laser welds 530a, 530b are arranged concentrically with respect to each other and are arranged on the outer region 514 of the convex disc 510. The load-bearing weld 530a is arranged concentrically outside the sealing weld 530b and covers a larger surface area than the sealing weld 530b. The load-bearing weld 530a is configured to prevent separation of the convex disc 510 from the collar 520. The sealing weld 530b is configured to provide a fluid-tight seal between the convex disc 510 and collar 520.

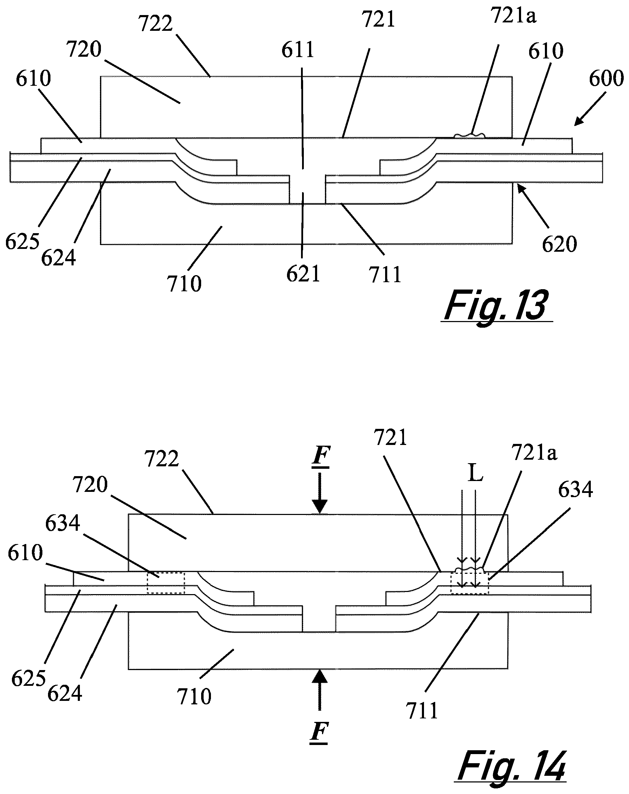

Referring to Figures 13 to 16 a third embodiment of an ostomy baseplate 600 comprises many of the same features as the first embodiment described above and as such only the differences between the two embodiments are described. In addition, like numerals are used to denote like features.

In this embodiment, the clamping surface 721 of the transfer plate 720 comprises a marking 721a provided in relief on the surface. In this embodiment, it is debossed into the surface but in others it could be embossed or comprise embossed and debossed parts. The marking 721a could represent any information that would be useful for the user, for example, branding, logos, instructions on how to use the baseplate, or product identifying information.

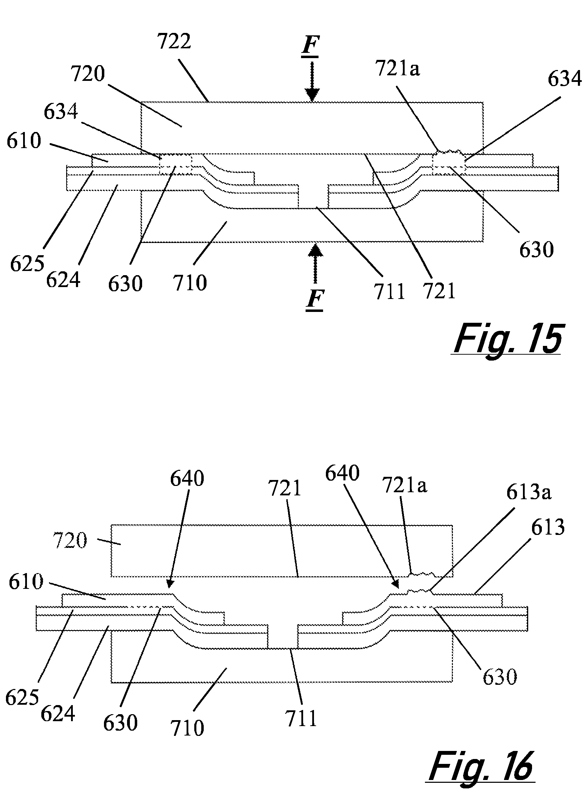

With reference to Figure 13, before the laser weld is formed, the distal surface 613 of the transfer plate 610 is flat. Then, as shown in Figure 14 and 15, during welding, the melt zone 634 expands to conform to the shape of the marking 721a. Then, once the weld is formed, as shown in Figure 16, a corresponding marking 613a is formed in relief on the distal surface 613 of the transfer plate 610. This is preferably in a position that is visible to the user during use. In this embodiment, this is facilitated by subsequent positioning of the coupling weld 640 on the inside of the laser weld as indicated in Figure 16.

The one or more embodiments are described above by way of example only. Many variations are possible without departing from the scope of protection afforded by the appended claims.

## Claims

1. A method of manufacturing a baseplate (100, 500, 600) configured to secure an ostomy appliance (300) to the body of a user, the method comprising providing a convex disc (110, 510, 610) and a collar (120, 520, 620), **characterised in that** the convex disc (110, 510, 610) is formed from a material that is more rigid than the collar (120, 520, 620) and the method comprises directing a laser beam onto the convex disc and collar to form a laser weld (130, 530a, 530b, 630) therebetween.

2. A method according to claim 1 wherein the method includes absorbing part of the laser beam into the convex disc (110, 510, 610) to cause melting of the convex disc (110, 510, 610) during formation of the laser weld (130, 530a, 530b, 630).

3. A method according to claim 2 wherein the method includes absorbing part of the laser beam into the collar (120, 520, 620) to cause melting of the collar (120, 520, 620) during formation of the laser weld (130, 530a, 530b, 630).

4. A method according to claim 2 or 3 wherein the convex disc (110, 510, 610) comprises a thermoplastics material which volumetrically absorbs the laser beam to cause melting of the convex disc (110, 510, 610).

5. A method according to any preceding claim wherein the laser beam has a wavelength of 1.2 to 2.2 µm.

6. A method according to claim 5 wherein the laser beam has a wavelength of 1.6 to 2.2 µm.

7. A method according to any preceding claim wherein the collar (120, 520, 620) is configured to secure the baseplate to the body of a user and comprises a stomal aperture (121, 521, 621).

8. A method according to claim 7 wherein the collar (120, 520, 620) comprises a proximal surface (112) configured to contact the body of a user in use and the proximal surface (112) comprises an adhesive (124, 624).

9. A method according to any preceding claim wherein the method comprises melting the convex disc (110, 510, 610) and/or collar (120, 520, 620) during formation of the laser weld (130, 530a, 530b, 630) wherein the part of the convex disc (110, 510, 610) and/or collar (120, 520, 620) that melts during formation of the laser weld (130, 530a, 530b, 630) is free from additives configured to absorb visible light.

10. A method according to any preceding claim wherein the method comprises clamping the convex disc (110, 510, 610) and collar (120, 520, 620) together during formation of the laser weld (130, 530a, 530b, 630).

11. A method according to claim 10 further comprising clamping the convex disc (110, 510, 610) and collar (120, 520, 620) between a support plate (410, 710) and a transfer plate (420, 720), wherein the transfer plate (420, 720) comprises a marking (721a) provided in relief on a clamping surface of the transfer plate (420, 720), and the method comprises clamping the transfer plate (420, 720) against the convex disc (110, 510, 610), and melting the convex disc (110, 510, 610) during formation of the laser weld (130, 530a, 530b, 630) such that a corresponding marking (613a) is formed in relief on a surface of the convex disc (110, 510, 610).

12. A method according to any preceding claim wherein the method comprises directing the laser beam onto the collar (120, 520, 620) via the convex disc (110, 510, 610) and melting substantially the entire thickness of the convex disc (110, 510, 610) during formation of the laser weld (130, 530a, 530b, 630).

13. A method of manufacturing an ostomy appliance comprising manufacturing a baseplate according to any of claims 1 to 12, providing a pouch configured to receive stomal output from the baseplate, and heat sealing the baseplate to the pouch.

14. A method of forming a body fitment component for a two-piece ostomy pouch, the method comprising manufacturing a baseplate (100, 500, 600) according to any of claims 1 to 12 and providing the baseplate (100, 500, 600) with a coupling element for coupling to a corresponding coupling element on a pouch.

15. A baseplate (100, 500, 600) for securing an ostomy appliance (300) to the body of a user comprising a convex disc (110, 510, 610), a collar (120, 520, 620), **characterised in that** the convex disc (110, 510, 610) is formed from a material that is more rigid than the collar (120, 520, 620), and the baseplate comprises a laser weld (130, 530a, 530b, 630) between the convex disc and collar (120, 520, 620).

## Patentansprüche

1. Verfahren zum Herstellen einer Grundplatte (100, 500, 600), die dazu ausgebildet ist, eine Ostomievorrichtung (300) am Körper eines Benutzers zu sichern, das Verfahren umfassend ein Bereitstellen einer konvexen Scheibe (110, 510, 610) und eines Kragens (120, 520, 620), **dadurch gekennzeichnet, dass** die konvexe Scheibe (110, 510, 610) aus einem Material gebildet ist, das steifer ist als der Kragen (120, 520, 620), und das Verfahren ein Ausrichten eines Laserstrahls auf die konvexe Scheibe und den Kragen umfasst, um dazwischen eine Laserschweißnaht (130, 530a, 530b, 630) zu bilden.

2. Verfahren nach Anspruch 1, wobei das Verfahren ein Absorbieren eines Teils des Laserstrahls in die konvexe Scheibe (110, 510, 610) umfasst, um ein Schmelzen der konvexen Scheibe (110, 510, 610) während des Bildens der Laserschweißnaht (130, 530a, 530b, 630) zu bewirken.

3. Verfahren nach Anspruch 2, wobei das Verfahren ein Absorbieren eines Teils des Laserstrahls in den Kragen (120, 520, 620) umfasst, um ein Schmelzen des Kragens (120, 520, 620) während des Bildens der Laserschweißnaht (130, 530a, 530b, 630) zu bewirken.

4. Verfahren nach Anspruch 2 oder 3, wobei die konvexe Scheibe (110, 510, 610) ein thermoplastisches Material umfasst, welches den Laserstrahl volumetrisch absorbiert, um ein Schmelzen der konvexen Scheibe (110, 510, 610) zu bewirken.

5. Verfahren nach einem der vorhergehenden Ansprüche, wobei der Laserstrahl eine Wellenlänge von 1,2 bis 2,2 µm aufweist.

6. Verfahren nach Anspruch 5, wobei der Laserstrahl eine Wellenlänge von 1,6 bis 2,2 µm aufweist.

7. Verfahren nach einem der vorhergehenden Ansprüche, wobei der Kragen (120, 520, 620) dazu ausgebildet ist, die Grundplatte am Körper eines Benutzers zu sichern, und eine Stomaöffnung (121, 521, 621) aufweist.

8. Verfahren nach Anspruch 7, wobei der Kragen (120, 520, 620) eine proximale Oberfläche (112) aufweist, die dazu ausgebildet ist, bei Gebrauch mit dem Körper eines Benutzers in Kontakt zu gelangen, und wobei die proximale Oberfläche (112) einen Klebstoff (124, 624) aufweist.

9. Verfahren nach einem der vorhergehenden Ansprüche, wobei das Verfahren Ein Schmelzen der konvexen Scheibe (110, 510, 610) und/oder des Kragens (120, 520, 620) während des Bildens der Laserschweißnaht (130, 530a, 530b, 630) umfasst, wobei der Teil der konvexen Scheibe (110, 510, 610) und/oder des Kragens (120, 520, 620), der während des Bildens der Laserschweißnaht (130, 530a, 530b, 630) schmilzt, frei von Additiven ist, die dazu ausgebildet sind, sichtbares Licht zu absorbieren.

10. Verfahren nach einem der vorhergehenden Ansprüche, wobei das Verfahren Ein Zusammenklemmen der konvexen Scheibe (110, 510, 610) und des Kragens (120, 520, 620) während des Bildens der Laserschweißnaht (130, 530a, 530b, 630) umfasst.

11. Verfahren nach Anspruch 10, weiterhin umfassend ein Zusammenklemmen der konvexen Scheibe (110, 510, 610) und des Kragens (120, 520, 620) zwischen einer Stützplatte (410, 710) und einer Transferplatte (420, 720), wobei die Transferplatte (420, 720) eine Markierung (721a) umfasst, die als Relief auf einer Klemmfläche der Transferplatte (420, 720) bereitgestellt ist, und das Verfahren ein Klemmen der Transferplatte (420, 720) gegen die konvexe Scheibe (110, 510, 610) und ein Schmelzen der konvexen Scheibe (110, 510, 610) während des Bildens der Laserschweißnaht (130, 530a, 530b, 630) umfasst, so dass eine entsprechende Markierung (613a) als Relief auf einer Oberfläche der konvexen Scheibe (110, 510, 610) gebildet wird.

12. Verfahren nach einem der vorhergehenden Ansprüche, wobei das Verfahren ein Ausrichten des Laserstrahls durch die konvexe Scheibe (110, 510, 610) auf den Kragen (120, 520, 620) und ein Schmelzen im Wesentlichen der gesamten Dicke der konvexen Scheibe (110, 510, 610) während des Bildens der Laserschweißnaht (130, 530a, 530b, 630) umfasst.

13. Verfahren zur Herstellung einer Ostomievorrichtung, umfassend ein Herstellen einer Grundplatte gemäß einem der Ansprüche 1 bis 12, ein Bereitstellen eines Beutels, der dazu ausgebildet ist, einen Stoma-Ausfluss von der Grundplatte aufzunehmen, und ein Heißsiegeln der Grundplatte an den Beutel.

14. Verfahren zum Bilden einer Körperanpassungskomponente für einen zweiteiligen Ostomiebeutel, wobei das Verfahren ein Herstellen einer Grundplatte (100, 500, 600) gemäß einem der Ansprüche 1 bis 12 und ein Bereitstellen der Grundplatte (100, 500, 600) mit einem Koppelelement zum Koppeln mit einem entsprechenden Koppelelement an einem Beutel umfasst.

15. Grundplatte (100, 500, 600) zum Befestigen einer Ostomievorrichtung (300) am Körper eines Benutzers, umfassend eine konvexe Scheibe (110, 510, 610) und einen Kragen (120, 520, 620), **dadurch gekennzeichnet, dass** die konvexe Scheibe (110, 510, 610) aus einem Material gebildet ist, das steifer ist als der Kragen (120, 520, 620), und die Grundplatte eine Laserschweißnaht (130, 530a, 530b, 630) zwischen der konvexen Scheibe und dem Kragen (120, 520, 620) aufweist.

## Revendications

1. Un procédé de fabrication d'une plaque (100, 500, 600) de base configurée pour assujettir un appareil (300) de stomie au corps d'un utilisateur, le procédé comprenant se procurer un disque (110, 510, 610) convexe et une rondelle (120, 520, 620), **caractérisé en ce que** le disque (110, 510, 610) convexe est en un matériau, qui est plus rigide que la rondelle (120, 520, 620) et le procédé comprend diriger un faisceau laser sur le disque convexe et sur la rondelle pour former une soudure (130, 530a, 530b, 630) laser entre eux.

2. Un procédé suivant la revendication 1, dans lequel le procédé comprend absorber une partie du faisceau laser dans le disque (110, 510, 610) convexe pour provoquer une fusion du disque (110, 510, 610) convexe pendant la formation de la soudure (130, 530a, 530b, 630) laser.

3. Un procédé suivant la revendication 2, dans lequel le procédé comprend absorber une partie du faisceau laser dans la rondelle (120, 520, 620) pour provoquer une fusion de la rondelle (120, 520, 620) pendant la formation de la soudure (130, 530a, 530b, 630) laser.

4. Un procédé suivant la revendication 2 ou 3, dans lequel le disque (110, 510, 610) convexe comprend une matière thermoplastique, qui absorbe volumétriquement le faisceau laser pour provoquer une fusion du disque (110, 510, 610) convexe.

5. Un procédé suivant l'une quelconque des revendications précédentes, dans lequel le faisceau laser a une longueur d'onde allant de 1,2 à 2,2 µm.

6. Un procédé suivant la revendication 5, dans lequel le faisceau laser a une longueur d'onde allant de 1,6 à 2,2 µm.

7. Un procédé suivant l'une quelconque des revendications précédentes, dans lequel la rondelle (120, 520, 620) est configurée pour assujettir la plaque de base au corps d'un utilisateur et comprend une ouverture (121, 521, 621) stomale.

8. Un procédé suivant la revendication 7, dans lequel la rondelle (120, 520, 620) comprend une surface (112) proximale configurée pour être en contact avec le corps d'un utilisateur en utilisation et la surface (112) proximale comprend un adhésif (124, 624).

9. Un procédé suivant l'une quelconque des revendications précédentes, dans lequel le procédé comprend faire fondre le disque (110, 510, 610) convexe et/ou la rondelle (120, 520, 620) pendant la formation de la soudure (130, 530a, 530b, 630) laser, dans lequel la partie du disque (110, 510, 610) convexe et/ou de la rondelle (120, 520, 620), qui fond pendant la formation de la soudure (130, 530a, 530b, 630) laser est exempte d'additif configuré pour absorber la lumière visible.

10. Un procédé suivant l'une quelconque des revendications précédentes, dans lequel le procédé comprend serrer ensemble le disque (110, 510, 610) convexe et la rondelle (120, 520, 620) pendant la formation de la soudure (130, 530a, 530b, 630) laser.

11. Un procédé suivant la revendication 10, comprenant, en outre, serrer le disque (110, 510, 610) convexe et la rondelle (120, 520, 620) entre une plaque (410, 710) de support et une plaque (420, 720) de transfert, dans lequel la plaque (420, 720) de transfert comprend un repère (721a) en relief sur une surface de serrage de la plaque (420, 720) de transfert et le procédé comprend serrer la plaque (420, 720) de transfert sur le disque (110, 510, 610) convexe et faire fondre le disque (110, 510, 610) convexe pendant la formation de la soudure (130, 530a, 530b, 630) laser, de manière à former un repère (613a) correspondant en relief sur une surface du disque (110, 510, 610) convexe.

12. Un procédé suivant l'une quelconque des revendications précédentes, dans lequel le procédé comprend diriger le faisceau laser sur la rondelle (120, 520, 620) par l'intermédiaire du disque (110, 510, 610) convexe et faire fondre sensiblement toute l'épaisseur du disque (110, 510, 610) convexe pendant la formation de la soudure (130, 530a, 530b, 630) laser.

13. Un procédé de fabrication d'un appareil de stomie, comprenant fabriquer une plaque de base suivant l'une quelconque des revendications 1 à 12, ménager un sac configuré pour recevoir la sortie stomale de la plaque de base et sceller à chaud la plaque de base au sac.

14. Un procédé de formation d'un composant d'ajustement au corps pour un sac de stomie en deux pièces, le procédé comprenant fabriquer une plaque (100, 500, 600) de base suivant l'une des revendications 1 à 12 et munir la plaque (100, 500, 600) de base d'un élément d'accouplement à un élément d'accouplement correspondant sur un sac.

15. Une plaque (100, 500, 600) de base d'assujettissement d'un appareil (300) de stomie au corps d'un utilisateur comprenant un disque (110, 510, 610) convexe, une rondelle (120, 520, 620), **caractérisée en ce que** le disque (110, 510, 610) convexe est en un matériau, qui est plus rigide que la rondelle (120, 520, 620) et la plaque de base comprend une soudure (130, 530a, 530b, 630) laser entre le disque convexe et la rondelle (120, 520, 620).
